# EUROPÄISCHE PATENTANMELDUNG

(11) **EP 3 117 843 A1**
(43) Veröffentlichungstag der Anmeldung: **18.01.2017**
(21) Anmeldenummer: 16020095.2
(22) Anmeldetag: 25.05.2009
(51) Int. Cl.: A61L 27/50, A61L 29/18, A61L 31/18

(54) **MEDIZINISCHES INSTRUMENT MIT RÖNTGEN-MARKER-PARTIKELN UND MR-MARKER**

(30) Priorität: 23.05.2008 DE 102008024976
(62) Teilanmeldung aus: 09749655.8
(71) Anmelder: MaRVis Medical GmbH, 50226 Frechen (DE)
(72) Erfinder: Pfeffer, Joachim, 52070 Aachen (DE); Düring, Klaus, 50226 Frechen (DE)
(74) Vertreter: Schüssler, Andrea

(57) **Zusammenfassung**

Medizinisches Instrument, das in einen menschlichen oder tierischen Körper einführbar ist, wobei das medizinische Instrument einen Instrumentenkörper aufweist, und der Instrumentenkörper zumindest einen schlecht elektrisch leitenden stabförmigen Körper aufweist, der aus einem Matrixwerkstoff und nicht-metallischen Filamenten ausgebildet ist. Das medizinische Instrument zeichnet sich entweder dadurch aus, dass der stabförmige Körper mit Röntgen-Marker-Partikeln dotiert ist, und das medizinische Instrument einen MR-Marker aufweist, oder dass der Instrumentenkörper im Oberflächenbereich mit einem immobilisierten aktiven MR-Marker versehen ist.

## Beschreibung

Die vorliegende Erfindung betrifft ein medizinisches Instrument. Insbesondere betrifft die vorliegende Erfindung ein medizinisches Instrument, das mittels der Magnetresonanztomographie detektierbar ist.

Aus der WO 2007/000148 A2 geht ein stabförmiger Körper, der zur Ausbildung medizinischer Instrumente, wie zum Beispiel Katheter oder Führungsdrähte für Katheter, dient, hervor. Dieser stabförmige Körper besteht aus einem oder mehreren Filamenten und einem nicht-ferromagnetischen Matrixwerkstoff, wobei der Matrixwerkstoff die Filamente umschließt. In dem Matrixwerkstoff ist eine Dotierung aus magnetresonanztomographische Artefakte erzeugenden Partikeln eingebracht.

Eine ausführliche Erläuterung der Magnetresonanztomographie (MRT-Magnetic-Resonance-Energing) ist im Internet unter http:/en.wikipedia.org/wiki/MRT erhältlich.

Aus der US 2003/0055449 A1 geht ein Ballonkatheter hervor, bei dem der Ballon aus einem Poymermaterial mit einem ferromagnetischen oder paramagnetischen Material ausgebildet ist, so dass er bei der Magnetresonanz sichtbar ist.

Aus der US 5,154,179 geht ein Katheter hervor, der beispielsweise aus einem extrudierten Kunststoffschlauch ausgebildet ist, wobei in dem Kunststoffmaterial des Kunststoffschlauches ferromagnetische Partikel enthalten sind. Dieser Katheter ist in der Magnetresonanztomographie sichtbar. Weiterhin wird vorgeschlagen einen solchen Katheter mit Material zu versehen, das für Röntgenstrahlung opak ist. Vorzugsweise werden als solche Röntgen-Marker Nicht-Eisenmaterialien verwendet.

Aus der DE 101 07 750 A1 geht ein Führungsdraht hervor, der für die Magnetresonanztomographie geeignet sein soll. Dieser Führungsdraht weist eine Seele aus einem metallischen Vorderteil auf. Zwischen einem äußeren Mantel und der Seele sind Seile aus einem nicht elektrisch leitenden Kunststoff angeordnet. Dieser Kunststoff soll mittels Glasfasern oder Kohlefasern verstärkt sein. Kohlefasern sind allerdings auch elektrische Leiter, so dass sie für die Magnetresonanztomographie nicht verwendet werden können.

Weiterhin sind aus der EP 1 206 945 A1 medizinische Arbeitsmittel bekannt, die mit paramagnetischen Metallverbindungen und/oder einem paramagnetischen Metall versetzt sind, so dass sie in einem Magnetresonanztomographieverfahren sichtbar sind.

Aus der WO 87/02893 sind polychelierende Stoffe für die Abbildungs- und Spektralerhöhung für die Magnetresonanztomographie offenbart. Diese Stoffe umfassen unterschiedliche Komplexe, in welchen Metall-Ionen, insbesondere Gadolinium-Ionen, immobilisiert sind.

In "Neue, radioaktiv markierte und Magnet-Resonanz-aktive Somatostatinanaloga zur besseren Diagnose und zielgerichteten Radionuklidtherapie von neuroendokrinen Tumoren", Daniel Storch, Inauguraldissertation, Basel, 2005, ist im Kapitel 1.6.1 die Relaxivität von Gadolinium(III)-Komplexen erläutert. Die paramagnetische Relaxation der Wassermoleküle, die sich in der Nähe des Gadolinium(III)-Ions befinden, entsteht durch die Dipol-Dipol-Interaktion zwischen dem Kernspin und dem fluktuierenden lokalen magnetischen Feld des Magnetresonanztomographiegerätes, verursacht durch die ungepaarten Elektronen. Das magnetische Feld um das paramagnetische Zentrum, also das Gadolinium(III)-Ion verschwindet mit zunehmendem Abstand. Darum ist es entscheidend, dass die Protonen in unmittelbare Nähe des Metalliones gebracht werden. Für Gadolinium(III)-Komplexe bedeutet dies, dass die Wassermoleküle in die erste Koordinationsphäre des Metallions zu bringen sind. Diese "inner-sphere" H₂O-Moleküle werden mit den umliegenden Wassermolekülen ausgetauscht und geben so den paramagnetischen Effekt weiter.

In der DE 100 40 381 C1 sind fluoralkylhaltige Komplexe mit Zuckerresten offenbart. Diese Komplexe können mit paramagnetischen Metallionen versehen sein, so dass sie als Kontrastmittel in der Magnetresonanztomographie dienen können. Diese Metallionen sind insbesondere die zwei- und dreiwertigen Ionen der Elemente der Ordnungszahlen 21-29, 42, 44 und 58-70. Geeignete Ionen sind beispielsweise das Chrom(III)-, Eisen(II)- , Kobalt(II)-, Nickel(II)-, Kupfer(II)-, Praseodym(III)-, Neodym(III)-, Samarium(III)- und Ytterbium(III)-lon. Wegen ihres starken magnetischen Moments sind besonders bevorzugt Gadolinium(III)-, Erbium(III)-, Dysprosium(III)-, Holmium(III)-, Erbium(III)-, Eisen(III)- und Mangan(II)-Ionen.

Aus der EP 1 818 054 A1 geht die Verwendung von Gadoliniumchelaten zur Markierung von Zellen hervor.

Aus der US 6,458,088 B1 geht ein für die Magnetresonanztomographie vorgesehener Führungsdraht hervor, der einen Glaskörper aufweist. Der Glaskörper ist mit einer Schutzschicht aus Polymermaterial versehen, die zusätzlich mit Fasern verstärkt sein kann. Das distale Ende des Führungsdrahtes kann aus einem Metallabschnitt ausgebildet sein, wie zum Beispiel Nitinol. Dieser Metallabschnitt sollte deutlich kürzer als die Wellenlänge des Magnetresonanzfeldes sein.

Aus der WO 2005/120598 A1 geht ein Katheter-Führungsdraht hervor, der einen PEEK-Kern aufweist. Dieser Kern ist beschichtet. Die Beschichtung ist mit einem Kontrastmittel versehen. Das Kontrastmittel ist ein Eisenpulver mit einer Korngröße von unter 10 µm.

In der WO 97/17622 ist ein medizinisches Instrument offenbart, das einen nicht elektrisch leitenden Körper aufweist, der mit einer ultradünnen Beschichtung aus elektrisch leitendem Material versehen ist, so dass das medizinische Instrument in einer Magnetresonanztomographie sichtbar ist, ohne dass das Bild zu sehr zu gestört wird.

Aus der WO 99/060920 A und der WO 2002/022186 A geht jeweils eine Beschichtung für ein medizinisches Instrument mit einem paramagnetischen Ion hervor, das in der Beschichtung komplexiert ist. Das paramagnetische Ion ist insbesondere Gadolinium. Diese Beschichtung ist in der Magnetresonanztomographie-Untersuchung sichtbar.

Der Erfindung liegt die Aufgabe zugrunde, ein medizinisches Instrument zu schaffen, das in einem menschlichen oder tierischen Körper einführbar ist und das sehr flexibel in einer Magnetresonanztomographie-Untersuchung einsetzbar ist.

Die Aufgabe wird durch ein medizinisches Instrument mit den Merkmalen des Anspruchs 1 oder des Anspruchs 2 gelöst. Vorteilhafte Ausgestaltungen sind in den Unteransprüchen angegeben.

Nach einem ersten Aspekt der vorliegenden Erfindung wird ein medizinisches Instrument vorgesehen, das in einem menschlichen oder tierischen Körper einführbar ist, wobei das medizinische Instrument einen Instrumentenkörper aufweist. Der Instrumentenkörper weist zumindest einen schlecht elektrisch leitenden stabförmigen Körper auf, der aus einem Matrixwerkstoff und nicht-metallischen Filamenten ausgebildet ist. Dieses medizinische Instrument zeichnet sich dadurch aus, dass der stabförmige Körper mit einem Röntgen-Marker dotiert ist, und das medizinische Instrument einen MR-Marker aufweist.

Durch das Vorsehen eines Röntgen-Markers und eines MR-Markers ist das medizinische Instrument sowohl in Röntgenuntersuchungen als auch der Magnetresonanztomographie sichtbar. Die Einbringung des Röntgen-Markers in das medizinische Instrument lässt sich einfach durch Verwendung eines entsprechend dotierten stabförmigen Körpers realisieren. Derartige stabförmige Körper können in unterschiedlichen Dotierungen als Massenprodukt günstig und mit einer exakten Dosierung der Marker-Partikel hergestellt werden. Bei der Herstellung eines medizinischen Instrumentes kann somit durch Verwendung des entsprechenden stabförmigen Körpers mit Röntgen-Marker die Sichtbarmachung des medizinischen Instrumentes in Röntgenuntersuchungen gewährleistet werden.

Das erfindungsgemäße medizinische Instrument nach einem zweiten Aspekt ist zum Einführen in einen menschlichen oder tierischen Körper ausgebildet, wobei es einen Instrumentenkörper mit einer Oberfläche aufweist, die mit dem menschlichen oder tierischen Körper in Kontakt kommen kann. Der Oberflächenbereich des Instrumentenkörpers ist mit immobilisierten aktiven MR-Markern versehen.

Aktive MR-Marker sind Marker, die mit den Protonen im Wasser- oder Fettmolekül interagieren und zu einer schnelleren Relaxation der dem Marker benachbarten Protonen nach deren induzierte Orientierung durch das angelegte Magnetfeld führen. Die durch die Markierung verursachte Reduzierung der Relaxationszeit bewirkt starke MRT-Signale, was zu einem entsprechend starken Kontrast in den hierdurch erzeugten Bildern führt.

Durch die Verwendung eines immobilisierten aktiven MR-Markers an der Oberfläche des Instrumentenkörpers in Verbindung mit zumindest einem mit einem Marker dotierten stabförmigen Körper wird auf einfache Art und Weise der hohe Kontrast eines aktiven MR-Markers bei der Magnetresonanztomographie und der vielseitige Anwendungsbereich passiver Marker kombiniert. Die passiven Marker können sowohl für Röntgen- als auch für Magnetresonanz-Untersuchungen ausgebildet sein. Vorzugsweise weist das medizinische Instrument mehrere stabförmige Körper auf, die unterschiedlich dotiert sind.

Medizinische Instrumente, die an ihrer Oberfläche mit aktiven MR-Markern versehen sind, besitzen einen sehr flexiblen Einsatzbereich bezüglich der in einer Magnetresonanztomographie-Untersuchung verwendeten Sequenzen und sind auch bei Magnetresonanztomographie-Untersuchungen mit unterschiedlichen Sequenzen gleichmäßig sichtbar.

Die aktiven MR-Marker umfassen ein Element oder eine Kombination von Elementen oder eine Verbindung eines Elements aus der Gruppe von Gadolinium, Cer, Praseodym, Neodym, Promethium, Samarium, Europium, Terbium, Dysprosium, Holmium, Erbium, Thulium, Ytterbium und Lutetium. Diese Elemente können als Ionen in einem Komplex gebunden sein. Sie können aber auch in Form von Salzen oder Legierungen vorliegen.

Besonders bevorzugt wird als aktiver MR-Marker Gadolinium verwendet. Dies ist vorzugsweise mittels eines Komplexes, insbesondere eines Chelatkomplexes, immobilisiert.

Die Komplexe können entweder kovalent auf der Oberfläche des Instrumentenkörpers gebunden oder in eine quellbare Beschichtung eingebettet sein, die auf der Oberfläche des Instrumentenkörpers ausgebildet ist.

Zwischen den Komplexen und der Oberfläche des Instrumentenkörpers können Spacer angeordnet sein, so dass der aktive MR-Marker mit Abstand zur Oberfläche des Instrumentenkörpers angeordnet ist. Hierdurch wird sicher gestellt, dass die Marker von Körperfluid umspült werden und sich ein Großteil der MR-Marker in unmittelbarer Nachbarschaft zu Protonen von Wasser- und/oder Fettmolekülen befindet.

Beim Vorsehen einer quellbaren Beschichtung, in welcher sich die MR-Marker befinden, wird beim Einführen des medizinischen Instrumentes in den menschlichen oder tierischen Körper Körperflüssigkeit von der quellbaren Beschichtung aufgenommen, so dass sich Protonen von Wassermolekülen eng an die MR-Marker anlagern, was zu der die Relaxationszeit verkürzenden Wechselwirkung führt.

Die Erfindung wird nachfolgend beispielhaft näher anhand der in den Zeichnungen dargestellten Ausführungsbeispielen erläutert. Die Zeichnungen zeigen:
- Figur 1: einen Führungsdraht gemäß einer ersten Ausführungsform der Erfindung in einem Querschnitt,
- Figur 2: einen Führungsdraht gemäß einer weiteren Ausführungsform der vorliegenden Erfindung in einem Querschnitt,
- Figur 3: eine Testanordnung mit mehreren Stäben, die mit unterschiedlichen Markern versehen sind,
- Figur 4a bis 4f: Bilder, die von der Testanordnung mittels Magnetresonanz- bzw. Computertomographie erstellt worden sind,
- Figur 5: einen Führungsdraht gemäß einer weiteren Ausführungsform der Erfindung in einem Querschnitt,
- Figur 6: einen Führungsdraht gemäß einer weiteren Ausführungsform der Erfindung in einem Längsschnitt, und
- Figur 7a bis 7e: Bilder, die von weiteren Testanordnungen mittels Magnetresonanzbzw. Computertomographie erstellt worden sind.

Die Erfindung wird nachfolgend beispielhaft anhand eines Führungsdrahts 1 für einen Katheter beschrieben. Der Führungsdraht 1 ist aus einem Material ausgebildet, das keine magnetresonanztomographischen Artefakte erzeugt. Ein derartiges Material ist beispielsweise eine Keramik oder ein Kunststoff wie PEEK, PEBAX, PE, PP, PU, Silikon, Polymilchsäurepolymere, aromatische Polyamide oder Gedächtniskunststoffe sein. Insbesondere ist der Kunststoff mit Fasern verstärkt. Hierbei kann neben den bereits genannten Kunststoffen als Matrixmaterial auch Epoxidharz verwendet werden. Die Fasern sind Glasfasern oder Keramikfasern oder Kevlar®-Fasern, Dacron, pflanzliche Fasern (z.B. Seide, Sisal, Hanf, etc.). Materialien, die keine magnetresonanztomographischen Artefakte erzeugen, dürfen keine elektrisch leitenden Abschnitte aufweisen. Die elektrisch leitenden Abschnitte sollen nicht länger als 15 cm sein und insbesondere nicht länger als 10 cm oder 5 cm sein. Deshalb ist es auch möglich elektrisch leitende Fasern, wie z.B. Kohlefasern bzw. Carbonfasern, oder elektrisch leitende Drähte zu verwenden, sofern sichergestellt ist, dass die Abschnitte ausreichend voneinander elektrisch isoliert sind. Sie dürfen nicht aus einem ferromagnetischen, paramagnetischen, ferrimagnetischen oder antiferromagnetischen Material ausgebildet sein.

Der Führungsdraht ist ein langgestreckter Körper mit kreisförmigem Querschnitt und einem Durchmesser von üblicherweise nicht mehr als 2 mm (zum Beispiel 0,7 mm). An seiner Oberfläche 2 sind auf dem Führungsdraht aktive MR-Marker 3 immobilisiert.

Aktive MR-Marker sind Marker, die mit einem Protonen enthaltenden Medium, wie zum Beispiel Wasser- oder Fettmolekülen, derart wechselwirken, dass sie zu einer schnelleren Relaxation der dem MR-Marker benachbarten Protonen nach deren induzierter Orientierung durch ein angelegtes Magnetfeld führen. Derartige MR-Marker weisen beispielsweise ein Element oder eine Kombination von Elementen oder eine Verbindung eines Elements aus der Gruppe von Gadolinium, Cer, Praseodym, Neodym, Promethium, Samarium, Europium, Terbium, Dysprosium, Holmium, Erbium, Thulium, Ytterbium, Lutetium auf. Diese Elemente sind vorzugsweise mittels eines Komplexes, insbesondere mittels eines Chelatkomplexes, immobilisiert. Sie können auch als Salze oder in Legierungen vorliegen.

Typische Chelatbildner sind EDTA (ethylenediaminetetraacetic acid) DTPA (diethylenetriaminepentaacetic acid) und DOTA (1,4,7,10-tetrazacyclododecane-N,N',",N"' tetraacetic acid).

Grundsätzlich sind als Komplexe chemische (u.a. Polylysine, Dendrimere) oder biologische Makromoleküle (Proteine, Zucker, u.a. Dextran) geeignet.

Im vorliegenden Ausführungsbeispiel sind die MR-Marker Gadolinium(III)-Chelatkomplexe, wobei die Chelatkomplexe mittels kovalenter Bindung an der Oberfläche 2 des Führungsdrahtes 1 gebunden sind. Vorzugsweise sind zwischen den Chelatkomplexen und der Oberfläche 2 Spacer-Moleküle vorgesehen, so dass die MR-Marker mit Abstand zur Oberfläche 2 angeordnet sind. Als Spacer-Molekül ist zum Beispiel Polyethylenglykol geeignet.

Die kovalente Bindung zwischen den Chelaten, Spacern und dem aus einem Polymer ausgebildeten Instrumentenkörper kann über Amino-, quaternäre Ammonium-, Hydroxyl-, Carboxy-, Sulfhydryl-, Sulfat-, Sulfonium-, Thiolgruppen, reaktive Stickstoffgruppen, etc. (jeweils für Chelatbildner und für Polymer) ausgebildet sein.

Der Führungsdraht 1 wird zum Einführen von Kathetern in Blutgefäße verwendet. Beim Einführen des Führungsdrahtes 1 im Blutgefäß kommt die Oberfläche 2 des Führungsdrahtes 1 mit dem Blut in Berührung. Die mit Abstand zur Oberfläche 2 angeordneten MR-Marker 3 werden von dem Blut umspült, so dass sich an einen Großteil der MR-Marker 3 Wassermoleküle anlagern. Die MR-Marker wechselwirken mit den Wassermolekülen derart, dass deren Relaxationszeit herabgesetzt wird. In einer Magnetresonanztomographie-Untersuchung erzeugen diese Wassermoleküle ein kontrastreiches Signal. Hierdurch wird der Führungsdraht in dem mittels der Magnetresonanztomographie erzeugten Bild gut sichtbar. Die an der Oberfläche 2 des Führungsdrahtes 1 immobilisierten aktiven MR-Marker 3 bewirken in allen bekannten Sequenzen (zum Beispiel T1-gewichtet, T2-gewichtet, Gradientenecho-Sequenz etc.) einen gleichmäßigen Kontrast. Bei herkömmlichen medizinischen Instrumenten, die mit passiven MR-Markern versehen sind (zum Beispiel WO 2007/000148 A2) ist es zwar auch möglich, dass diese gut mittels der Magnetresonanztomographie detektierbar sind, jedoch bewirken die passiven MR-Marker eine Störung der Feldlinien, die bei unterschiedlichen Sequenzen unterschiedlich stark ausgeprägt ist, was oftmals dazu führt, dass bei bestimmten Sequenzen das Bild derart stark bzw. so wenig gestört ist, dass es für die medizinische Untersuchung nicht brauchbar ist. Deshalb können medizinische Instrumente, die mit passiven MR-Markern versehen sind, nicht bei allen Sequenzen eingesetzt werden oder die Konzentration der passiven MR-Marker ist so gering bzw. so hoch, dass sie bei gewissen Sequenzen nicht mehr sichtbar sind bzw. zu starke Signale ergeben, die die umgebenden Strukturen überdecken.

Medizinische Instrumente, die wie der oben beschriebene Führungsdraht an ihrer Oberfläche mit aktiven MR-Markern versehen sind, besitzen gegenüber Instrumenten mit passiven MR-Markern wegen des anderen zugrundeliegenden physikalischen Effekts einen wesentlich flexibleren Einsatzbereich bezüglich der Sequenzen und sind auch bei Magnetresonanztomographie-Untersuchungen mit unterschiedlichen Sequenzen gleichmäßig sichtbar.

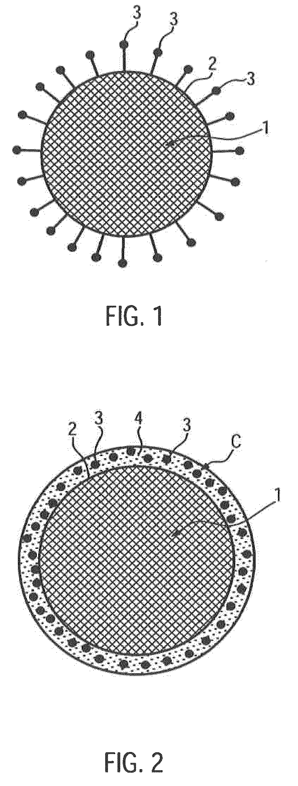

Figur 2 zeigt ein zweites Ausführungsbeispiel eines erfindungsgemäßen Instrumentes, das wiederum ein Führungsdraht 1 mit einer Oberfläche 2 ist. Der Körper des Führungsdrahtes ist so wie der Körper des Führungsdrahtes nach dem ersten Ausführungsbeispiel ausgebildet. Die Oberfläche 2 ist mit einer quellbaren Beschichtung 4 versehen. Solche quellbaren Beschichtungen sind zum Beispiel aus Polyvinylpyrrolidon (PVP) ausgebildet. Solche quellbaren Beschichtungen sind u.a. unter dem Handelsnamen Colidon bzw. Kollidon von der BASF AG erhältlich.

In die quellbare Beschichtung sind aktive MR-Marker eingebettet. Beispielsweise wird als MR-Marker ein Gadolinium(III)-Chelatkomplex verwendet.

Beim Eintauchen in eine wässrige Umgebung oder in eine Fettumgebung nimmt die quellbare Beschichtung 4 Wassermoleküle bzw. Fettmoleküle auf, so dass sich die Wasser- bzw. Fettmoleküle an die aktiven MR-Marker anlagern. Die MR-Marker wechselwirken mit den in Wasser- bzw. Fettmolekülen enthaltenen Protonen, so dass deren Relaxationszeit reduziert wird und sie in einer Magnetresonanztomographie-Untersuchung sichtbar sind.

Auch diese Ausführungsform des Führungsdrahtes kann mittels beliebiger Sequenzen in der Magnetresonanztomographie detektiert werden. Dieser Führungsdraht ist deshalb bezüglich der Magnetresonanztomographie sehr flexibel einsetzbar.

Die aktiven MR-Marker sind in elementarer oder freier Form in der Regel toxisch. Befinden sich die aktiven Marker in Komplexen gebunden, so sind sie in der Regel für den menschlichen und tierischen Körper gut verträglich. Je höher die Bindungskonstante im Chelatkomplex ist, desto geringer ist die Dissoziation des MR-Markers aus dem Komplexbildner und damit auch das Risiko, dass elementarer MR-Marker frei in die Körperflüssigkeiten gelangen kann. Bei der Erfindung sind die aktiven MR-Marker an dem jeweiligen medizinischen Instrument immobilisiert, so dass sie nach der Untersuchung zusammen mit dem Instrument aus dem menschlichen oder tierischen Körper entfernt werden. Die Gefahr einer toxischen Wirkung ist somit minimal.

Die Erfindung ist oben anhand zweier Führungsdrähte erläutert worden. Die Erfindung ist jedoch nicht auf Führungsdrähte beschränkt. Im Rahmen der Erfindung können beliebige in tierische oder menschliche Körper einführbare Instrumente erfindungsgemäß ausgestaltet werden, indem im Oberflächenbereich des Instrumentenkörpers aktive MR-Marker derartig immobilisiert werden, dass sie mit den Protonen im körpereigenen Medium wechselwirken können. Derartige Instrumente sind zum Beispiel Katheter, Stents oder Implantate. Der Instrumentenkörper ist vorzugsweise aus einem Material ausgebildet, das keine oder nur geringe magnetresonanztomographische Artefakte erzeugt, so dass der Kontrast überwiegend durch die im Oberflächenbereich angeordneten aktiven MR-Marker verursacht wird. Solche Materialien sind vorzugsweise Kunststoffe, insbesondere glasfaserverstärkte Kunststoffe. Sie können jedoch auch Keramiken und Verbundmaterial aus Keramik und Kunststoff sein.

Nach einem weiteren Aspekt der vorliegenden Erfindung sind die medizinischen Instrumente sowohl mit MR-Markern als auch mit Röntgen-Markern versehen. Als MR-Marker werden vorzugsweise aktive MR-Marker in der oben erläuterten Weise eingesetzt. Es ist jedoch auch möglich, passive MR-Marker zu verwenden. Passive MR-Marker sind paramagnetische, ferromagnetische, ferrimagnetische und antiferromagnetische Metalle, Metalllegierungen und Metallverbindungen. Sie sind vorzugsweise als Partikel in eine Kunststoffmatrix eingebettet. Die passiven MR-Marker sind vorzugsweise folgende Metalle oder Metallverbindungen: Cobalt (Co), Nickel (Ni), Molybdän (Mo), Zirkonium (Zr), Titan (Ti). Mangan (Mn), Rubidium (Rb), Aluminium (Al), Palladium (Pd), Platin (Pt), Chrom (Cr) oder Chromdioxid (CrO₂), und insbesondere Eisen (Fe) und Eisenoxid (FeO, Fe₂O₃, Fe₃O₄). Die Konzentration der passiven MR-Marker ist so zu wählen, dass sie bei den gewünschten Sequenzen sichtbar sind, das medizinische Instrument in zumindest einer MR-Sequenz gut abbilden, aber die Abbildung des umgebenden Körpergewebes dabei nicht überlagern oder stören. Die an der Oberfläche angeordneten aktiven MR-Marker werden jedoch bevorzugt, da sie wesentlich flexibler einsetzbar sind.

Als Röntgen-Marker werden hingegen folgende Metalle oder andere Elemente Barium (Ba), Wolfram (W), Tantal (Ta), Osmium (Os), Praseodym (Pr), Platin (Pt), Gold (Au) und Blei (Pb) eingesetzt. Diese Elemente können in elementarer Form oder auch in Verbindungen, wie z.B. Bariumsulfat, als Röntgen-Marker verwendet werden.

Die Röntgen-Marker beeinflussen in der Regel die Bildgebung bei einem magnetresonanztomographischen Verfahren kaum. Sie sind jedoch in Röntgenuntersuchungen, z.B. der Computertomographie oder Durchleuchtungen, mittels Röntgenstrahlung gut erkennbar.

Einige Marker können grundsätzlich sowohl als Röntgen-Marker als auch als passiver MR-Marker verwendet werden, wobei die bildgebende Funktion jeweils von der Konzentration abhängt. Wie es unten noch näher ausgeführt wird, erzeugt Eisen sowohl bei einer Magnetresonanztomographie als auch bei einer Röntgenuntersuchung Bildsignale. Jedoch sind die für die Röntgenuntersuchung notwendigen Eisen-Konzentrationen so hoch, dass hierdurch das Bild bei der Magnetresonanztomographie gestört wird. Marker, die sowohl als Röntgen-Marker als auch als MR-Marker verwendbar sind, werden in einer solchen Konzentration eingesetzt, dass sie weder die Magnetresonanztomographie noch die Röntgenuntersuchung stören. In der Regel ist die Konzentration dieser Marker so eingestellt, dass sie nur bei der Magnetresonanztomographie ein Bildsignal erzeugen und bei der Röntgen-Untersuchung kaum sichtbar sind. Bei Platin ist die Situation ähnlich, aber hier ist der Unterschied in der Wirkung bei den beiden bildgebenden Verfahren nicht so ausgeprägt.

Der Röntgen-Marker ist aus Partikeln ausgebildet, die in einem stabförmigen Körper eingebettet sind. Der stabförmige Körper ist wiederum Bestandteil des medizinischen Instrumentes, das mehrere solche stabförmigen Körper umfassen kann, die mit den gleichen oder auch unterschiedlichen einschließlich passiven MR-Markern versehen sein können. Ein solcher stabförmiger Körper ist vorzugsweise ausgebildet, wie es in der WO 2007/000148 A2 beschrieben ist. Auf dieses Dokument wird diesbezüglich Bezug genommen.

Der stabförmige Körper ist aus einem Matrixwerkstoff ausgebildet, der nicht-metallische Filamente und die Partikel des jeweiligen Markers umschließt. Der Matrixwerkstoff ist vorzugsweise ein Kunststoff, wie z,B. Epoxidharz, PEEK, PEBAX, PE, PP, PU, Silikon, Polymilchsäurepolymere. Die Filamente sind bspw. Glasfasern, Keramikfasern, Dacron, Kevlar® oder pflanzliche Fasern (z.B. Seide, Sisal, Hanf, etc.).

Der stabförmige Körper ist schlecht elektrisch leitend ausgebildet. Die Partikel der Marker können grundsätzlich gut elektrisch leitend sein (z.B. Eisen- oder Platin-Partikel). Sie sind jedoch in einer solchen Konzentration vorzusehen, dass sie durch das Matrixmaterial voneinander isoliert sind und zumindest keinen elektrischen Leiter ausbilden, der länger als 15 cm und vorzugsweise nicht länger als 10 cm bzw. 5 cm ist.

Die Verwendung derartiger stabförmiger Körper, die in der Regel einen Durchmesser von 0,1 mm bis 0,7 mm und vorzugsweise von 0,1 mm bis 0,3 mm aufweisen, erlaubt die einfache Herstellung medizinischer Instrumente, wobei das medizinische Instrument auf einfache Weise mit unterschiedlichen Markern ausgebildet werden kann, indem es aus unterschiedlich dotierten stabförmigen Körpern ausgebildet wird. Die stabförmigen Körper können in ein weiteres übergeordnetes Matrixmaterial zur Ausbildung des medizinischen Instrumentes eingebettet werden. Sie können jedoch auch zur Ausbildung eines medizinischen Instrumentes geflochten werden.

Ein medizinisches Instrument mit zumindest einem Röntgen-Marker und zumindest einem MR-Marker kann somit sowohl bei einer Röntgenuntersuchung als auch bei einer Magnetresonanzuntersuchung eingesetzt werden und ist jeweils gut sichtbar, ohne dass die Bildgebung durch einen der beiden Marker gestört wird.

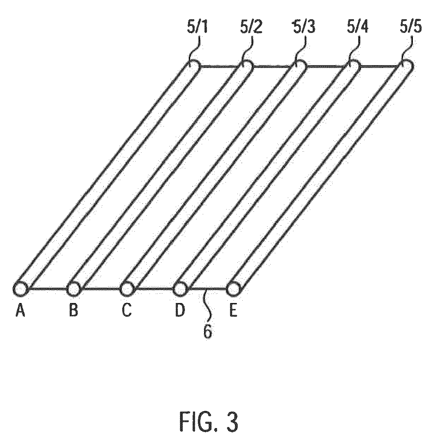

Figur 3 zeigt eine Testanordnung zum Testen unterschiedlicher Marker in unterschiedlichen Bildgebungsverfahren. Die Testanordnung weist fünf Teststäbe 5 auf, die auf einer Kunststoffplatte 6 angeordnet sind. Die Teststäbe sind jeweils aus einem zwei-Komponenten-Epoxidharz hergestellt worden. Einer der Teststäbe 5/1 besteht ausschließlich aus dem Epoxidharz. Zwei der Teststäbe 5/2, 5/3 sind mit einem Wolfram-Pulver und zwei weitere Teststäbe 5/4, 5/5 mit einem Eisen-Pulver dotiert. Das Eisen-Pulver wird unter der Handelsbezeichnung Eisenrothipuran von der Firma Roth unter der Nummer 3718.1 vertrieben. Es besitzt eine Reinheit von zumindest 99,5 %. Die Korngröße liegt im Bereich von 4 bis 6 µm. Das Wolfram-Pulver ist Wolfram fein gepulvert 99+ von der Firma Merck KGaA, das unter der Nummer 1.12406.0100 vertrieben wird. Es besitzt eine Reinheit von zumindest 99,0 %. Die Korngröße ist kleiner 20 µm. Das Wolfram-Pulver ist paramagnetisch. Der Teststab 5/2 weist einen Anteil von 10 Gew% Wolfram-Pulver auf. Der Teststab 5/3 weist einen Anteil von 1 Gew% Wolfram-Pulver auf. Der Teststab 5/4 weist einen Anteil von 10 Gew% Eisen-Pulver auf. Der Teststab 5/5 weist einen Anteil von 1 Gew% Eisen-Pulver auf.

Diese Testanordnung wurde in einer mit Wasser (37°C) gefüllten Wanne derart angeordnet, dass sich unterhalb der Testanordnung zumindest eine Wasserschicht von 5 mm und oberhalb der Testanordnung eine Wasserschicht von zumindest 25 mm befand.

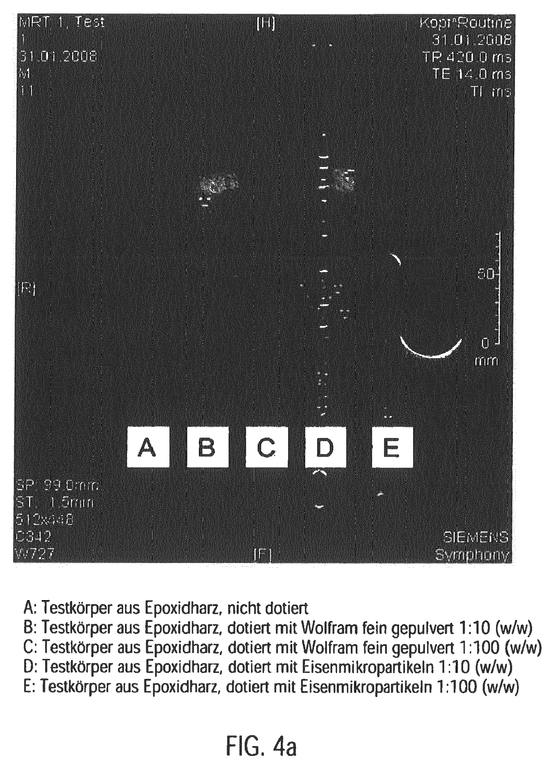

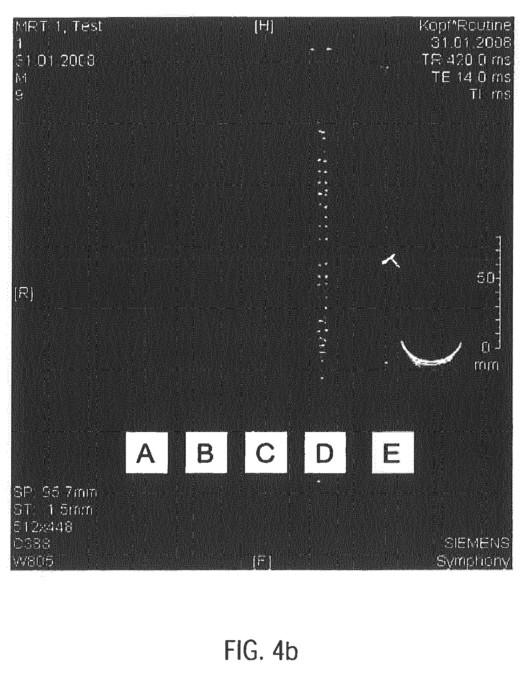

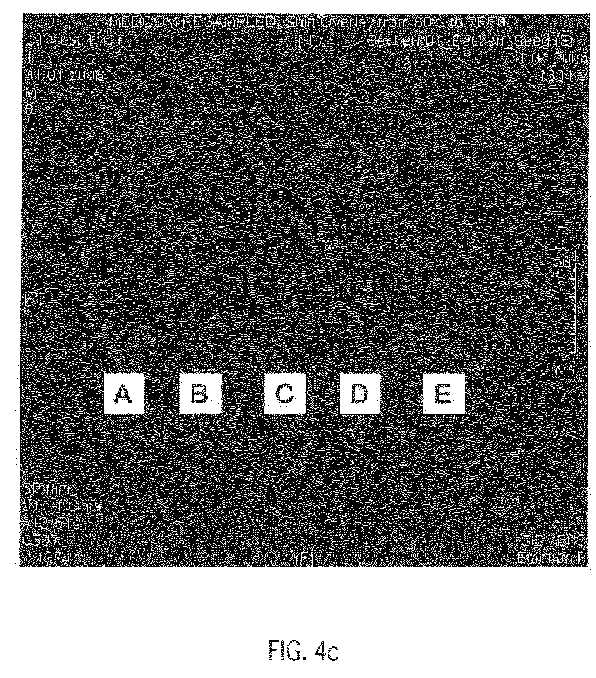

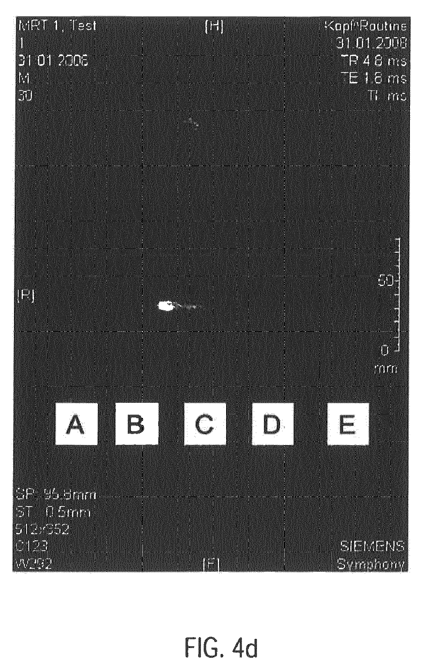

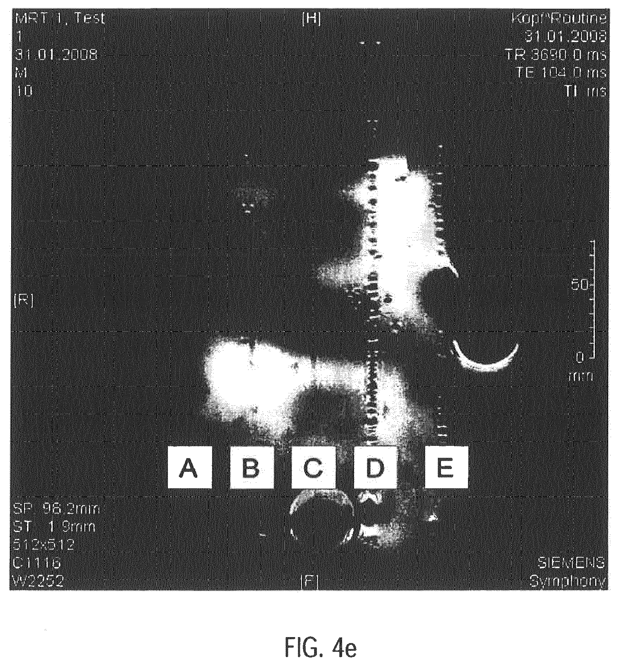

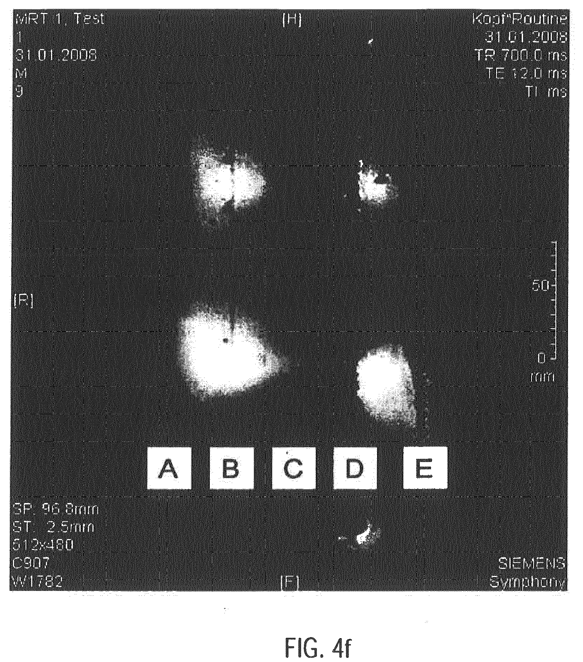

Diese Testanordnung wurde einer Magnetresonanztomographie mit einer T1-gewichteten Sequenz (Fig. 4a, 4b), einer Gradientenecho EPI-Sequenz (Fig. 4d), einer T2-gewichteten Sequenz (Fig. 4e) und einer Gradientenecho-Sequenz (Fig. 4f) unterzogen. Weiterhin wurde die Testanordnung einer Röntgenuntersuchung (CT) unterzogen (Fig. 4c).

Aus den Bildern ergibt sich klar, dass die Eisenpartikel auch in vergleichsweise niedrigen Konzentrationen bei einer Magnetresonanztomographie erhebliche Artefakte verursachen, die das Bild in der Umgebung des eisenhaltigen Bereiches derart stark stören, dass es für eine Analyse unbrauchbar ist. Dies gilt insbesondere für die Magnetresonanzuntersuchung mittels der Gradientenecho-Sequenz (Fig. 4f).

Wolfram, das eine wesentlich größere Ordnungszahl als Eisen besitzt, ist hingegen in den Magnetresonanz-Untersuchungen kaum sichtbar, da die Teststäbe 5/2 und 5/3 keinen stärkeren Kontrast verursachen als der Teststab 5/1, der keinerlei Dotierung aufweist. Der Teststab 5/2 mit einem Anteil von 10 Gew% Wolfram-Pulver ist in der Röntgen-Untersuchung (Fig. 4c) sehr gut sichtbar. Selbst der mit sehr geringer Wolfram-Dotierung versehene Teststab 5/3 ist in der Röntgen-Untersuchung noch erkennbar.

Grundsätzlich kann festgestellt werden, dass die Elemente der Röntgen-Marker in der Regel eine größere Ordnungszahl als die Elemente der MR-Marker aufweisen, wobei es jedoch auch einen überschneidenden Bereich gibt. Die bevorzugten passiven MR-Marker weisen mit Ausnahme von Platin (Ordnungszahl 78) eine Ordnungszahl von nicht mehr als 46 (Palladium) auf. Die bevorzugten Röntgen-Marker weisen hingegen eine Ordnungszahl von zumindest 56 (Barium) auf.

Hierdurch wird ein medizinisches Instrument erhalten, das sowohl bei einer Magnetresonanztomographie als auch bei einer Computertomographie sichtbar ist und zu keinen Störungen im Bild führt.

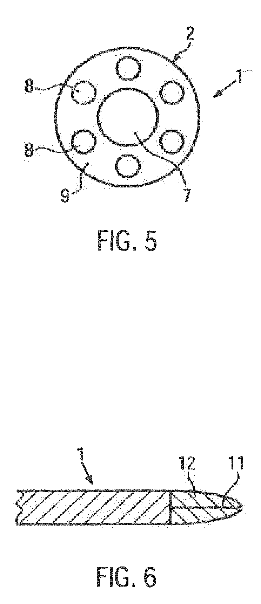

Figur 5 zeigt ein weiteres Beispiel eines erfindungsgemäßen medizinischen Instrumentes, das ein Führungsdraht 1 ist. Dieser Führungsdraht 1 weist sieben stabförmige Körper 7, 8 auf. Im Zentrum des Führungsdrahtes 1 ist ein zentraler stabförmiger Körper 7 angeordnet. Sechs radiale stabförmige Körper 8 sind um den zentralen stabförmigen Körper 7 gleichmäßig verteilt angeordnet. Alle stabförmigen Körper 7, 8 sind in einer Hüllmatrix 9 eingebettet. Die Oberfläche der Hüllmatrix 9 bildet die Oberfläche des Führungsdrahtes 1.

Die stabförmigen Körper 7, 8 sind, wie es oben erläutert ist, aus einem Matrixwerkstoff ausgebildet, der nicht-metallische Filamente enthält. Die obige Beschreibung der stabförmigen Körper gilt auch für die stabförmigen Körper 7, 8, sofern nachfolgend nichts anderes erläutert wird.

Der zentrale stabförmige Körper 7 weist einen größeren Durchmesser als die radialen stabförmigen Körper 8 auf. Hierdurch ist der zentrale stabförmige Körper 7 steifer als die radialen stabförmigen Körper 8. Da der zentrale stabförmige Körper 7 in der Mitte des Führungsdrahtes 1 angeordnet ist, wirkt sich seine höhere Steifigkeit nicht so sehr auf die Biegesteifigkeit des gesamten medizinischen Instrumentes wie die radialen stabförmigen Körper 8 aus, da er auf der Biegelinie des medizinischen Instrumentes angeordnet ist. Die radialen stabförmigen Körper 8 sind flexibler, weshalb sie die Biegesteifigkeit des medizinischen Instrumentes auch nicht zu stark beeinflussen. Hiermit wird ein medizinisches Instrument mit einer geeigneten Flexibilität erzielt.

Die in Figur 5 dargestellte Ausführungsform ist sehr vorteilhaft, da sie einen sehr dünnen Führungsdraht mit hoher Festigkeit und Flexibilität ergibt und aufgrund der radialen Anordnung der radialen stabförmigen Körper 8 weist der Führungsdraht 1 eine hohe Torsionssteifigkeit auf.

Weiterhin kann die Festigkeit und Flexibilität des medizinischen Instrumentes durch eine veränderte Anzahl der stabförmigen Körper und auch durch eine andere Anordnung, beispielsweise ohne zentralen stabförmigen Körper, verändert werden. Die Flexibilität des Führungsdrahtes ist eine wesentliche Eigenschaft und individuell an unterschiedliche Anwendungen anzupassen. Die Flexibilität des Führungsdrahtes kann durch Verändern des Durchmessers des zentralen stabförmigen Körpers und/oder der radialen stabförmigen Körper und durch Verändern der Zusammensetzung der Hüllmatrix variiert werden. Damit das medizinische Instrument die gewünschte Festigkeit und Flexibilität aufweist, ist es zweckmäßig, dass alle stabförmigen Körper vollständig von der Hüllmatrix umhüllt sind.

Die radialen stabförmigen Körper 8 können parallel zum zentralen stabförmigen Körper 7 verlaufen. Sie können jedoch auch in einer schraubenförmigen Anordnung um den zentralen stabförmigen Körper 7 angeordnet sein.

Der zentrale stabförmige Körper weist einen Durchmesser von 0,1 bis 0,4 mm und vorzugsweise von etwa 0,2 bis 0,3 mm auf. Der zentrale stabförmige Körper ist beispielsweise mit Wolfram-Nanopartikeln (Partikelgröße ca. 40-50 nm) dotiert.

Der Anteil der Wolfram-Partikel beträgt 50 Gew% am Matrixwerkstoff des stabförmigen Körpers. Im vorliegenden Ausführungsbeispiel werden die übrigen 50 Gew% von einem Epoxidharz beigetragen. Zusätzlich weist der stabförmige Körper Glasfasern auf.

Es hat sich gezeigt, dass die Wolfram-Nanopartikel bei der Herstellung des stabförmigen Körpers in vorteilhafter Weise die Fließfähigkeit des Epoxidharzes beeinflusst haben. Die nicht-dotierten stabförmigen Körper werden zur Verbesserung der Fließfähigkeit unter Beimischung von Aerosilen extrudiert. Bei Verwendung von Wolfram-Nanopartikel ist es nicht notwendig derartige Aerosile dem Epoxidharz beizumischen. Es hat sich gezeigt, dass je kleiner die Partikel sind, desto besser ist die Viskosität des Epoxidharzes.

Bei einem hohen Anteil an Wolfram-Partikeln wirken diese sowohl als Röntgen-Marker als auch als MR-Marker. Das Gewichtsverhältnis der Wolfram-Partikel zum Matrixwerkstoff sollte zumindest 1:2 bis 2:1 betragen. Je höher der Anteil der Wolfram-Partikel ist, desto besser wirken sie als MR-Marker. Diese Wirkung als MR-Marker hängt auch von der Größe des stabförmigen Körpers und damit der absoluten Menge der Wolfram-Partikel und der Partikelgröße der Wolfram-Partikel ab. So sind Wolfram-Partikel mit eine Größe von einigen µm bis zu etwa 20 µm kaum als MR-Marker geeignet, wie es oben anhand der Bilder 4a, 4b und 4d bis 4f erläutert wird. Je kleiner die Wolfram-Partikel sind, desto stärker wird ihre Wirkung als MR-Marker. Es hat sich gezeigt, dass das Gewichtsverhältnis der Wolfram-Partikel zum Matrixwerkstoff bis zu einem Bereich 2:1 bis 3:1 eingestellt werden kann.

Die radialen stabförmigen Körper 8 weisen einen Durchmesser von 0,10 bis 0,25 mm und vorzugsweise von 0,15 bis 0,20 mm auf. Lediglich ein einziger der radialen stabförmigen Körper 8 ist im vorliegenden Ausführungsbeispiel mit Fe₃O₄-Partikeln dotiert. Die Partikel besitzen eine Partikelgröße von etwa 40-50 nm. Die Partikel sollten nicht größer als 100 nm und vorzugsweise nicht größer als 60 nm sein. Im dotierten radialen stabförmigen Körper 8 kommen auf einem Gewichtsteil Fe₃O₄-Partikel ca. 10 bis 30 und bevorzugt 20-25 Gewichtsteile Matrixwerkstoff, der hier wieder vorzugsweise Epoxidharz ist. Die Fe₃O₄-Partikel sind passive MR-Marker.

Im Rahmen der Erfindung ist es selbstverständlich auch möglich, die stabförmigen Körper mit anderen Passivmarkern, anderen Konzentrationen und anderen Partikelgrößen zu dotieren. Es können auch mehr als zwei stabförmige Körper mit einem Marker, vorzugsweise mit unterschiedlichen Markern, dotiert sein. Die Anzahl, die Anordnungen und die Durchmesser der stabförmigen Körper können auch variieren.

In einem stabförmigen Körper können auch mehrere unterschiedliche Marker vorgesehen sein.

Im Rahmen der vorliegenden Erfindung ist es auch möglich, diesen Führungsdraht an der Oberfläche mit einer der oben beschriebenen Beschichtungen mit einem aktiven MR-Marker zu versehen.

Die Hüllmatrix 9 ist ein thermoplastisches Elastomer, vorzugsweise Polyurethan, insbesondere Tecoflex^{™}, bzw. Mediprene^{®}

Mediprene^{®} ist ein thermoplastisches Elastomer, das vor allem für medizinische Zwecke verwendet wird. Mediprene wird von der VTC Elastoteknik AB, Schweden, angeboten. Unter Mediprene^{®} wird Mediprene^{®} TO 34007 verstanden, was ein thermoplastisches Elastomer aus SEBS (Styrol-Ethylen-Butylen-Styrol-Elastomer) ist.

Das in Figur 5 gezeigte medizinische Instrument wird vorzugsweise durch Coextrusion der stabförmigen Körper und der Hüllmatrix hergestellt.

Die Verwendung unterschiedlich dotierter stabförmiger Körper ist nicht auf Führungsdrähte beschränkt. Unterschiedlich dotierte stabförmige Körper können auch bei anderen medizinischen Instrumenten, wie z.B. Kathetern, Stents oder Implantaten eingesetzt werden.

Vorzugsweise ist ein Führungsdraht 1 gemäß einer der obigen Ausführungsformen mit einer flexiblen Spitze 10 versehen (Figur 6). Die flexible Spitze 10 ist aus einem axialen Nylonfaden 11 und einem Polyurethankörper 12 ausgebildet. Diese flexible Spitze 10 wird durch schrittweises Beschichten des Nylonfadens hergestellt, so dass die flexible Spitze 10 als stumpfe Spitze geformt werden kann. Die flexible Spitze ist mittels einer Klebeverbindung mit einer Stirnfläche des Führungsdrahtes 1 verbunden. Vorzugsweise ist die flexible Spitze 10 mit einer der oben beschriebenen passiven Dotierungen dotiert und/oder mit einem aktiven Marker beschichtet.

Die Stirnfläche des Führungsdrahtes 1 und die korrespondierende Kontaktfläche der flexiblen Spitze 10 sind vorzugsweise konusförmig geschliffen, so dass die Berührungsfläche zwischen dem Führungsdraht 1 und der flexiblen Spitze 10 vergrößert ist.

Die flexible Spitze 10 kann auch durch Erhitzen der beiden Kontaktflächen mit dem Führungsdraht 1 verbunden werden. Es ist auch möglich, die flexible Spitze 10 mit einem chemischen Lösungsmittel (z.B. in solution grade Polyurethan) anzulösen und so mit dem Führungsdraht 1 zu verbinden. Ein geeignetes Lösungsmittel ist z.B. THF, wenn Polyurethan als Material für die flexible Spitze 10 verwendet wird. Anstelle von Polyurethan kann das Material für die flexible Spitze 10 auch Epoxidharz, PEEK, PEBAX, PE, PP, Silikon Polymilchsäure oder Mediprene^{®} verwendet werden. Der axiale Polymerfaden kann auch aus anderen Materialien, z.B. PEEK, PEBAX, PE, PP, Silikon oder Polymilchsäure ausgebildet werden. Die flexible Spitze kann auch ohne einen axialen Faden ausgebildet sein.

Der Nylonfaden ist vorzugsweise mit einem Marker dotiert. Er kann mit einem anderen Marker als das übrige Material der flexiblen Spitze 10 dotiert sein. Ist kein Faden vorhanden, so kann das Material für die flexible Spitze mit einem Marker dotiert werden.

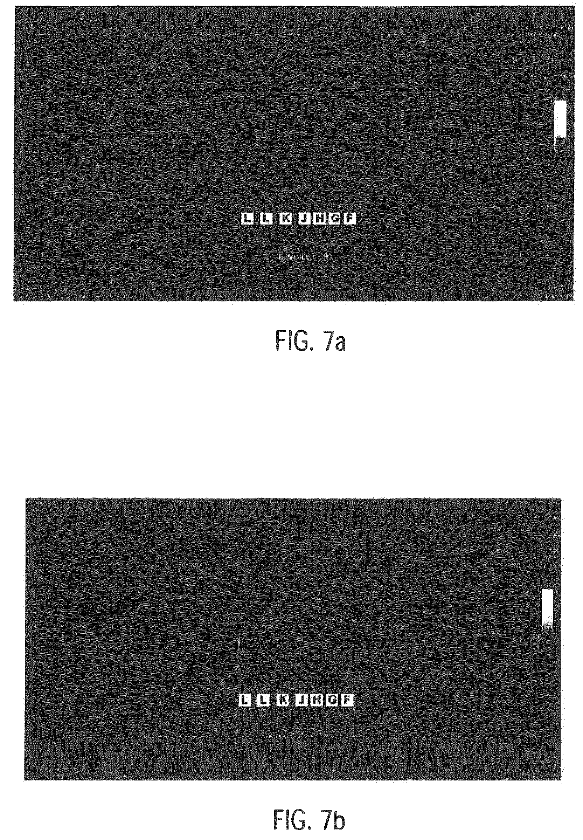

Figur 7a bis 7e zeigen weitere Testanordnungen, die mittels Magnetresonanz- bzw. Röntgentomographie erstellt worden sind.

Bei dieser Testanordnung wurden einerseits stabförmige Körper und andererseits Führungsdrähte untersucht, die sich wiederum in Wasser befinden.

Die stabförmigen Körper bestehen grundsätzlich aus Epoxidharz mit Glasfasern. Hierbei wurden folgende unterschiedliche stabförmige Körper untersucht:
(F) Durchmesser 0,17 mm; keine Dotierung
(G)Durchmesser 0,17 mm; mit Fe₃O₄-Nanopartikeln dotiert; das Gewichtsverhältnis zwischen Dotierung und Epoxidharz beträgt 1:20
(H)Durchmesser 0,27 mm; mit Wolfram-Nanopartikeln dotiert; Gewichtsverhältnis der Dotierung zu Epoxidharz 1:1
(J) Durchmesser 0,27 mm; mit Wolfram-Nanopartikeln dotiert; Gewichtsverhältnis der Dotierung zu Epoxidharz 2:1

Die untersuchten Führungsdrähte 1 weisen grundsätzlich den in Figur 5 gezeigten und anhand von Figur 5 beschriebenen Aufbau auf, wobei der zentrale stabförmige Körper 7 einen Durchmesser von 0,27 mm besitzt und mit Wolfram-Nanopartikeln dotiert ist. Die radialen stabförmigen Körper 8 besitzen einen Durchmesser von 0,17 mm. Fünf radiale stabförmige Körper 8 sind nicht dotiert. Einer der radialen stabförmigen Körper 8 ist mit Fe₃O₄-Nanopartikeln dotiert.

Folgende Führungsdrähte wurden untersucht:
(K) Hüllmatrix aus Polyurethan; Dotierung des zentralen stabförmigen Körpers aus Wolfram-Nanopartikeln im Gewichtsverhältnis 1:1 zum Epoxidharz, ein mit Fe₃O₄ dotierter radialer stabförmiger Körper 8, wobei das Gewichtsverhältnis der Dotierung zum Epoxidharz 1:20 beträgt;
(L) Hüllmatrix aus Mediprene^{®}; Dotierung des zentralen stabförmigen Körpers aus Wolfram-Nanopartikeln im Gewichtsverhältnis 2:1 zum Epoxidharz, ein mit Fe₃O₄ dotierter radialer stabförmiger Körper 8, wobei das Gewichtsverhältnis der Dotierung zum Epoxidharz 1:20 beträgt;

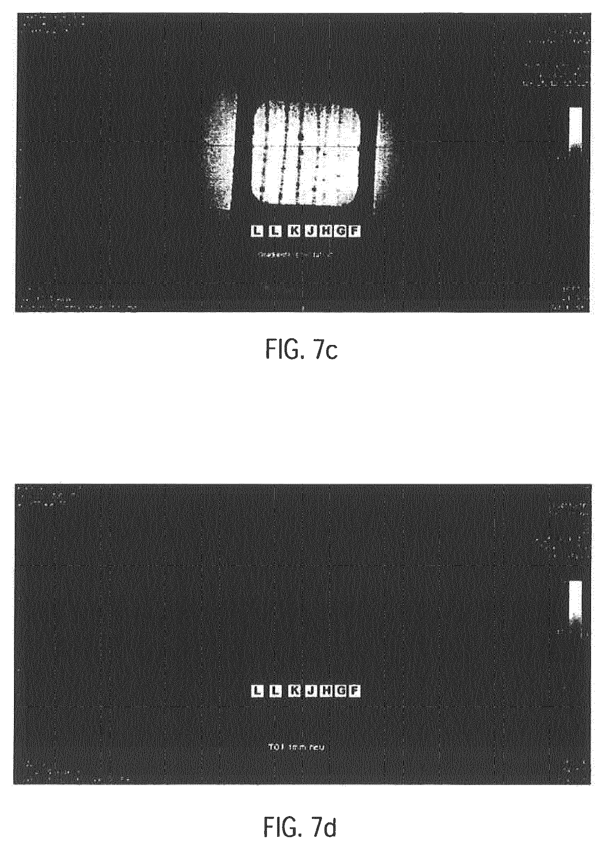

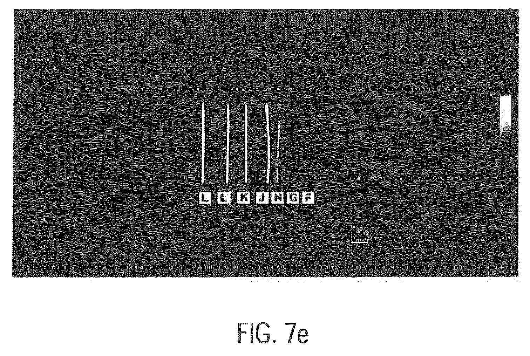

In Figur 7a ist eine T1-gewichtete MRT-Sequenz, in Figur 7b eine T2-gewichtete MRT-Sequenz, in Figur 7c eine MRT-Gradientenecho-Sequenz und in Figur 7d eine MRT-Angio-TOF-Sequenz gezeigt. In Figur 7e ist eine computertomographische Abbildung der stabförmigen Körper und der Führungsdrähte gezeigt.

Der nicht dotierte stabförmige Körper F ist in kaum einem der Bilder sichtbar. Bei der Magnetresonanztomographie sind teilweise sehr kontrastarme Spuren aufgrund der Verdrängung des Wassers in der Testanordnung sichtbar.

Der mit Fe₃O₄ dotierte radiale stabförmige Körper ist in der Magnetresonaztomographie mit unterschiedlichem Kontrast sichtbar. Bei der MRT-Gradientenecho-Sequenz und der MRT-Angio-TOF-Sequenz ist der Kontrast hoch und bei den beiden T1- und T2-gewichteten MRT-Sequenzen ist der Kontrast gering. Der mit Wolfram-Nanopartikeln dotierte stabförmige Körper H hat bei der Magnetresonanztomographie ähnliche Ergebnisse gezeigt, wobei die Kontraste bei den beiden T1- und T2-gewichteten MRT-Sequenzen besser als die des stabförmigen Körpers G sind. Weiterhin erzeugt der stabförmige Körper H auch bei der Computertomographie (Röntgenuntersuchung) einen hervorragenden Kontrast.

Ein solcher stabförmiger Körper, der mit Wolfram-Nanopartikeln (Partikelgröße kleiner 100 nm, vorzugsweise kleiner 60 nm) dotiert ist, stellt einen eigenen, selbständigen Erfindungsgedanken dar, da alleine die Verwendung eines solchen stabförmigen Körpers in einem medizinischen Instrument die Sichtbarmachung des medizinischen Instrumentes sowohl in einer Röntgen- als auch in einer Magnetresonanzuntersuchung bewirkt. Mit anderen Markern können zum Teil bessere Kontraste erzielt werden, so dass eine Kombination mit weiteren Markern nach wie vor sinnvoll aber nicht unbedingt notwendig ist. Wolfram-Nanopartikel besitzen auch den Vorteil, dass sie in einer vorbestimmten Konzentration im stabförmigen Körper sowohl bei einer Röntgen- als auch bei einer MR-Untersuchung einen guten Kontrast erzeugen. Eisenpartikel sind grundsätzlich auch geeignet sowohl bei einer Röntgen- als auch bei einer MR-Untersuchung einen Kontrast zu erzeugen. Jedoch besteht bei Eisenpartikeln das Problem, dass sie bei höheren Konzentrationen starke Artefakte erzeugen, die das Bild in einer größeren Umgebung stark stören. Bei geringen für die Magnetresonanztomographie geeigneten Konzentrationen sind die Eisenpartikel wiederum nicht bei einer Röntgenuntersuchung sichtbar.

Weiterhin kann man anhand der Figuren 7a bis 7d erkennen, dass sowohl die dotierten stabförmigen Körper als auch die Führungsdrähte, die dotierte stabförmige Körper enthalten, sämtlich in den untersuchten MRT-Sequenzen gut sichtbar sind.

### Bezugszeichenliste:

- 1: Führungsdraht
- 2: Oberfläche
- 3: MR-Marker
- 4: quellbare Beschichtung
- 5: Teststab
- 6: Kunststoffplatte
- 7: zentraler stabförmiger Körper
- 8: radialer stabförmiger Körper
- 9: Hüllmatrix
- 10: flexible Spitze
- 11: Nylonfaden
- 12: Polyurethankörper

## Patentansprüche

1. Medizinisches Instrument, das in einen menschlichen oder tierischen Körper einführbar ist,
wobei das medizinische Instrument einen Instrumentenkörper aufweist, und der Instrumentenkörper zumindest einen schlecht elektrisch leitenden stabförmigen Körper aufweist, der aus einem Matrixwerkstoff und nicht-metallischen Filamenten ausgebildet ist, wobei der stabförmige Körper mit zumindest einem passiven MR-Marker dotiert ist, und
wobei der Instrumentenkörper eine Hüllmatrix aufweist, in die der zumindest eine stabförmige Körper eingebettet ist und wobei der Instrumentenkörper im Oberflächenbereich mit zumindest einem immobilisierten aktiven MR-Marker versehen ist und das medizinische Instrument zumindest einen Röntgenmarker aufweist.

2. Medizinisches Instrument nach Anspruch 1,
**dadurch gekennzeichnet,**
**dass** der stabförmige Körper mit Röntgen-Marker-Partikeln dotiert ist.

3. Medizinisches Instrument nach Anspruch 1 oder 2,
**dadurch gekennzeichnet,**
**dass** der stabförmige Körper einen Durchmesser von 0,1 mm bis 0,7 mm und vorzugsweise von 0,1 mm bis 0,3 mm aufweist.

4. Medizinisches Instrument nach einem der Ansprüche 1 bis 3,
**dadurch gekennzeichnet,**
**dass** der stabförmige Körper mit Wolfram-Partikel dotiert ist, die im stabförmigen Körper in einem Verhältnis von zumindest 50 Gew.% zum Matrixwerkstoff des stabförmigen Körpers enthalten sind, so dass die Wolfram-Partikel sowohl einen MR-Marker als auch einen Röntgen-Marker bilden.

5. Medizinisches Instrument nach einem der Ansprüche 1 bis 4,
**dadurch gekennzeichnet,**
**dass** das medizinische Instrument zumindest einen weiteren stabförmigen Körper aufweist, der mit einem weiteren Marker und insbesondere mit einem passiven MR-Marker dotiert ist.

6. Medizinisches Instrument nach einem der Ansprüche 1 bis 5,
**dadurch gekennzeichnet,**
**dass** der zumindest eine stabförmige Körper mit zumindest zwei unterschiedlichen Markern dotiert ist.

7. Medizinisches Instrument nach einem der Ansprüche 2 bis 6,
**dadurch gekennzeichnet,**
**dass** die aktiven MR-Marker mittels Komplexen, insbesondere mittels Chelatkomplexen, im Oberflächenbereich immobilisiert sind,wobei die Komplexe vorzugsweise entweder kovalent am Instrumentenkörper gebunden sind oder in eine quellbare Beschichtung, die auf der Oberfläche des Instrumentenkörpers ausgebildet ist, eingebettet sind, und die aktiven MR-Marker ein Element oder eine Kombination von Elementen oder Verbindungen der Elemente aus der Gruppe von Gadolinium, Cer, Praseodym, Neodym, Promethium, Samarium, Europium, Terbium, Dysprosium, Holmium, Erbium, Thulium, Ytterbium, Lutetium, aufweisen.

8. Medizinisches Instrument nach einem der Ansprüche 1 bis 7,
**dadurch gekennzeichnet,**
**dass** das medizinische Instrument keine lang gestreckten, elektrisch leitenden Abschnitte aufweist und aus einem nicht ferromagnetischem Material ausgebildet ist.

9. Medizinisches Instrument nach einem der Ansprüche 1 bis 9,
**dadurch gekennzeichnet,**
**dass** die Röntgen-Marker eines oder mehrere der folgenden Elemente bzw. Verbindungen mit einem oder mehreren der folgenden Elemente, wie Barium (Ba), Wolfram (W), Tantal (Ta), Osmium (Os), Praseodym (Pr), Platin (Pt), Gold (Au), Blei (Pb) aufweisen.

10. Medizinisches Instrument nach einem der Ansprüche 1 bis 9,
**dadurch gekennzeichnet,**
**dass** der MR-Marker ein passiver MR-Marker ist und aus einem paramagnetischen, ferromagnetischen, ferrimagnetischen und antiferromagnetischen Metall, einer Metalllegierung oder Metallverbindung ausgebildet ist.

11. Medizinisches Instrument nach Anspruch 10,
**dadurch gekennzeichnet,**
**dass** der passive MR-Marker ein Metall oder eine Metalllegierung oder eine Metallverbindung mit Cobalt (Co), Nickel (Ni), Molybdän (Mo), Zirkonium (Zr), Titan (Ti). Mangan (Mn), Rubidium (Rb), Aluminium (Al), Palladium (Pd), Platin (Pt), Chrom (Cr) oder Eisen (Fe) umfasst.

12. Medizinisches Instrument nach einem der Ansprüche 1 bis 11,
**dadurch gekennzeichnet,**
**dass** es mehrere MR-Marker aufweist, die auf unterschiedliche Sequenzen, wie z.B. T1-gewichtet, T2-gewichtet oder Gradientenecho, optimiert sind.

13. Medizinisches Instrument nach einem der Ansprüche 1 bis 12,
**dadurch gekennzeichnet,**
**dass** der Instrumentenkörper ein aus der Hüllmatrix und einem oder mehreren stabförmigen Körpern hergestellter Körper ist, wobei die Hüllmatrix aus einem thermoplastischen Elastomer ausgebildet ist.

14. Medizinisches Instrument nach einem der Ansprüche 1 bis 13,
**dadurch gekennzeichnet,**
**dass** das medizinische Instrument ein Katheter, ein Führungsdraht für einen Katheter, ein Stent oder ein Implantat ist.

15. Verfahren zum Detektieren eines medizinischen Instruments in einem menschlichen oder tierischen Körper, wobei
in den menschlichen oder tierischen Körper ein medizinisches Instrument gemäß einem der Ansprüche 1 bis 14 eingeführt wird und mittels Magnetresonanztomographie und/oder Röntgentomographie detektiert wird.
